# EUROPEAN PATENT SPECIFICATION

(11) **EP 4 070 316 B1**
(45) Date of publication and mention of the grant of the patent: **10.12.2025**
(21) Application number: 20825174.4
(22) Date of filing: 27.11.2020
(51) Int. Cl.: G16B 5/00, G16B 20/00, G16B 50/20

(54) **PRIORITISING BIOLOGICAL TARGETS**
PRIORISIERUNG BIOLOGISCHER TARGETS
HIÉRARCHISATION DE CIBLES BIOLOGIQUES

(30) Priority: 03.12.2019 US 201962942958 P
(43) Date of publication of application: 12.10.2022
(73) Proprietor: BenevolentAI Technology Limited, London Greater London W1T 5HD (GB)
(72) Inventor: BOLLERMAN, Thomas Joseph, New York 10803 (US)
(74) Representative: Gill Jennings & Every LLP
(86) International application number: PCT/GB2020/053061
(87) International publication number: WO 2021/111113

(56) References cited:
- US-B2- 10 453 553
- FAILLI MARIO ET AL: "Prioritizing target-disease associations with novel safety and efficacy scoring methods", SCIENTIFIC REPORTS, vol. 9, no. 1, 8 July 2019 (2019-07-08), pages 1 - 11, XP055778528, DOI: 10.1038/s41598-019-46293-7
- JOUHYUN JEON ET AL: "A systematic approach to identify novel cancer drug targets using machine learning, inhibitor design and high-throughput screening", GENOME MEDICINE,, vol. 6, no. 7, 30 July 2014 (2014-07-30), pages 57, XP021193909, ISSN: 1756-994X, DOI: 10.1186/S13073-014-0057-7
- MEHLA KUSUM ET AL: "Tapping intoSalmonella typhimuriumLT2 genome in a quest to explore its therapeutic arsenal: A metabolic network modeling approach", BIOMEDICINE AND PHARMACOTHERAPY, ELSEVIER, FR, vol. 86, 7 December 2016 (2016-12-07), pages 57 - 66, XP029884806, ISSN: 0753-3322, DOI: 10.1016/J.BIOPHA.2016.11.129

## Description

The present application relates to systems and methods for prioritising biological targets. The presently disclosed techniques find particular application in the fields of biochemistry and drug discovery where a biological target having certain characteristics may be required.

### Background

In the field of drug discovery, there is a need to identify suitable biological targets such as genes, nucleic acid sequences, proteins, amino acid sequences, protein complexes, or biological pathways for the treatment of diseases. Typically, potentially suitable biological targets are reviewed by scientific experts in the field who manually review tables of data relating to the targets and rank or otherwise prioritise them according to required criteria. For example, a scientist might manually review data relating to the extent and rate of side effects associated with the biological targets. Other categories to review and take account of may include druggability, other safety aspects, and whether or not there is a known association between a target and the successful treatment of a disease. This process of manual review is time consuming and expensive and may affect results by virtue of human bias or error. The document Jeon et al., Genome Medicine, 2014, 6:57, teaches a method for identifying and prioritizing drug targets using machine learning.

Accordingly, there is a need for an improved technique for identifying suitable biological targets that does not require a user to manually review biological target data.

The embodiments described below are not limited to implementations which solve any or all of the disadvantages of the known approaches described above.

### Summary

This Summary is provided to introduce a selection of concepts in a simplified form that are further described below in the Detailed Description. This Summary is not intended to identify key features or essential features of the claimed subject matter, nor is it intended to be used to determine the scope of the claimed subject matter.

In a first aspect, the present disclosure provides a computer-implemented method of prioritising biological targets as set out in appended claim 1, the method comprising: receiving a selection of classes of one or more categories; for each of a plurality of biological targets, determining an extent of alignment of the biological target to each selected class; prioritising the biological targets based on the extents of alignment; and outputting a representation of one or more prioritised biological targets.

The classes of the categories represent values or value ranges of the categories. Optionally, the selected classes of one of the categories are not mutually adjacent. Optionally, the selection of classes comprises at least two classes of the same category. The categories represent properties of the biological targets. Optionally, the method comprises receiving a user input comprising the selection of classes of the one or more categories. The extent of alignment between a biological target and a selected class comprises a likelihood of the biological target falling within the selected class. Optionally, the likelihood corresponds to a distribution normalised across all classes of the same category. Optionally, the method comprises determining the extents of alignment from one or more data sources. Optionally, the method comprises aggregating the extents of alignment from classifications based on respective data sources. The method comprises determining the extents of alignment using a trained machine learning classifier. The biological targets comprise genes, nucleic acid sequences, proteins, amino acid sequences, protein complexes, and/or biological pathways. Optionally, prioritising the biological targets comprises identifying biological targets that match the user input by applying a minimum required extent of alignment for each selected class. Optionally, the method comprises determining confidence metrics for the extents of alignment and optionally ranking the biological targets that match the user input based on the confidence metrics. Optionally, the method comprises determining the confidence metrics using a machine learning technique. Optionally, prioritising the biological targets comprises ranking the biological targets based on their extents of alignment to the selected classes. Optionally, the user input comprises an indication of relative importance of the categories and prioritising the biological targets comprises using the indication of relative importance. Optionally, the method comprises outputting a representation of the biological targets that match the user input. Optionally, the method comprises outputting a representation of the ranking. Optionally, the method comprises outputting a representation of the confidence metrics. Optionally, the method comprises providing a graphical user interface as an input and/or output tool. The method comprises providing a user input tool to enable to a user to generate a manual tagging command to override at least part of the output, the manual tagging command specifying whether or not one of the biological targets falls within one of the classes. The method comprises training the classifier based on the manual tagging command and optionally using the override command to augment a set of training data.

In a second aspect, the present disclosure provides a computer-readable medium storing code that, when executed by a computer, causes the computer to perform the method of any previous claim, as set out in appended claim 14.

In a third aspect, the present disclosure provides a system for prioritising biological targets as set out in appended claim 15, the system comprising: an input module configured to receive a selection of classes of one or more categories; an analysis module configured, for each of a plurality of biological targets, to determine an extent of alignment of the biological target to each selected class; a prioritisation module configured to prioritise the biological targets based on the extents of alignment; and an output module configured to output a representation of one or more prioritised biological targets.

The methods described herein may be performed by software in machine readable form on a tangible storage medium e.g. in the form of a computer program comprising computer program code means adapted to perform all the steps of any of the methods described herein when the program is run on a computer and where the computer program may be embodied on a computer readable medium. Examples of tangible (or non-transitory) storage media include disks, thumb drives, memory cards etc. and do not include propagated signals. The software can be suitable for execution on a parallel processor or a serial processor such that the method steps may be carried out in any suitable order, or simultaneously.

This application acknowledges that firmware and software can be valuable, separately tradable commodities. It is intended to encompass software, which runs on or controls "dumb" or standard hardware, to carry out the desired functions. It is also intended to encompass software which "describes" or defines the configuration of hardware, such as HDL (hardware description language) software, as is used for designing silicon chips, or for configuring universal programmable chips, to carry out desired functions.

The preferred features may be combined as appropriate, as would be apparent to a skilled person, and may be combined with any of the aspects of the invention.

### Brief Description of the Drawings

Embodiments of the invention will be described, by way of example, with reference to the following drawings, in which:
Figure 1 is a block diagram of a system for prioritising biological targets according to an embodiment of the invention;
Figure 2 is a flow chart of a method that may be carried out by the system of Figure 1 according to an embodiment of the invention;
Figure 3 is a block diagram of an analysis module of the system showing optional features;
Figure 4 is a block diagram of example data sources that may be used by the system;
Figure 5 is a block diagram of a prioritisation module of the system showing optional features;
Figure 6 is a block diagram of an example implementation of the system;
Figure 7 is a block diagram of a variation of the system according to another embodiment of the invention; and
Figure 8 is a block diagram of a computer suitable for implementing embodiments of the invention.

Common reference numerals are used throughout the figures to indicate similar features.

### Detailed Description

Embodiments of the present invention are described below by way of example only. These examples represent the best ways of putting the invention into practice that are currently known to the Applicant although they are not the only ways in which this could be achieved. The description sets forth the functions of the example and the sequence of steps for constructing and operating the example. However, the same or equivalent functions and sequences may be accomplished by different examples.

In the field of biochemistry, the task of developing new treatments for diseases often involves attempting to identify suitable biological targets such as genes, nucleic acid sequences, proteins, amino acid sequences, protein complexes, or biological pathways with which drugs may interact. For the avoidance of doubt, in this document 'nucleic acid sequences' include deoxyribonucleic acids (DNA), including genes, as well as ribonucleic acids (RNA), and 'biological targets' include biological molecules, complexes or pathways that may be targeted by a drug for the treatment of a disease. In order to identify biological targets that are suitable from among a large number of potential candidates, an assessment of their characteristics and a process of decision making as to which candidates meet a set of desired criteria may be carried out. Depending on the context and the purpose for which a biological target is required, the desired characteristics may span multiple categories such as ligandability, safety, and therapeutic evidence which must all be taken into account, and as a result the search is required to consider multiple properties of the candidates at once. In traditional approaches, this complex analysis is carried out manually by a scientist reviewing data relating to potential biological targets and sifting the candidates for potential matches to the desired characteristics. In cases involving multiple categories and large numbers of potential biological targets, such manual analysis is time consuming and tends to create delays and increased costs of the process of developing new treatments for diseases.

The inventor has appreciated that there is a need for a system that can remove the burden of manually reviewing potential biological targets from the scientist and assist the process by automatically generating an output of biological targets that have been prioritised in a rational manner based on user specified criteria.

A system according to the invention for automatically prioritising biological targets is associated with a range of advantages. Not only does such a system save time, but it also removes the potential hazard of human bias in decision making which could limit or skew results. As such, it is likely to produce results that may be missed by a manual review of the available information. Furthermore, an automated system is able to review larger data sources and in a different way to a human, which also increases the likelihood of producing results that may not be found by a human expert.

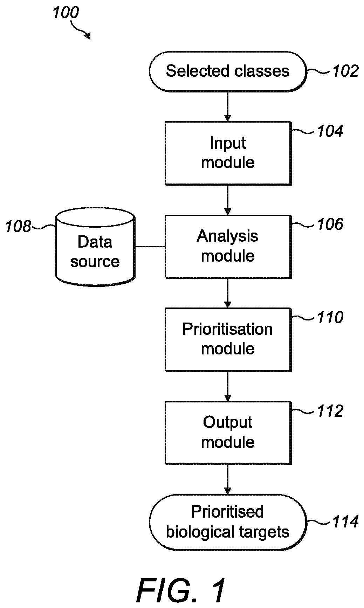

Figure 1 shows a system 100 for prioritising biological targets based on a user input according to an embodiment of the invention. It is to be appreciated that in other embodiments, biological targets may be based on predetermined or automatically generated criteria, rather than being based on a user input. In the embodiment of Figure 1, the system 100 is configured to receive a user input 102 at an input module 104 of the system 100. The user input 102 relates to required characteristics of a biological target which may comprise values or value ranges expressed in terms of allowable classes within various categories. The categories may represent physical, chemical or biological properties or other classifications or categorisations of the biological target. Other categorisations of the biological target may represent considerations such as how well known the target is for being associated with a particular disease. The allowable classes of these categories may represent allowable values or value ranges of these categories that the user requires. In this example, the input module 104 may suitably comprise a graphical user interface configured to receive user selections of allowable classes across one or more categories, where the categories may also be user selected. The values of some categories such as solubility may be numerical while the values of other categories may be expressed in words such as 'safe' or 'unsafe'. By specifying allowable classes of one or more categories, the user is able to indicate to the system the desired characteristics of the biological target that is required. In cases where the user selects allowable classes of multiple categories, a complex analysis taking into account multiple categories may be carried out automatically thereby removing the burden of manual analysis from the user.

A non-limiting list of example categories includes the following:
- Ligandability - An assessment of the probability that a small molecule modulator that interacts effectively with the biological target exists or can be created.
- Safety - An assessment of the probability that modulating the target may lead to serious clinical adverse events.
- Therapeutic Evidence - An assessment of the probability that modulating the target is already known to treat a related disease.
- Biological Rationale - An assessment of the probability that the dysregulation of the target causes disease.
- Target Expression - A measure of whether a target is expressed in relevant tissue/cell types and/or is differentially expressed in relevant healthy versus diseased tissue/cell types.
- Stratifiability - A measure of whether a target is distinctively expressed within between or different patient endotypes, which can be defined by a clinical characteristic or latent variable. For example, a latent variable may provide a measure of whether a target is expressed distinctively between different patient endotypes, which may be defined by clinical or biological data. In this case, differences in expression occur between disease subgroups and within a particular disease endotype the target may be expressed consistently within predefined bounds. In another example, a latent variable may provide a measure of whether a target is expressed distinctively within a patient endotype, which may be defined by clinical or biological data. In this case, differences in expression occur within a disease subgroup such that the target is expressed in multiple ways within a single endotype of interest.

The system 100 comprises an analysis module 106 configured to determine, for each of a plurality of biological targets, an extent of alignment of the biological target to each selected class of the user input. The extent of alignment of a biological target to a selected class provides a measure of the likelihood of the biological target falling within that selected class. As such, the determination of the extends of alignment provides insight into how well each biological target matches the criteria specified by the user. The extents of alignment may for example be expressed numerically by percentages or probabilities, or may in other suitable examples be expressed in words such as 'high likelihood' or 'low likelihood'. In cases where the extents of alignment are expressed in terms of numerical probabilities, the probabilities may correspond to a distribution such as a probability distribution normalised across all classes of the same category. Large numbers such as hundreds, thousands or hundreds of thousands of biological targets may be interrogated by the analysis module 106 using data from one or more data sources 108.

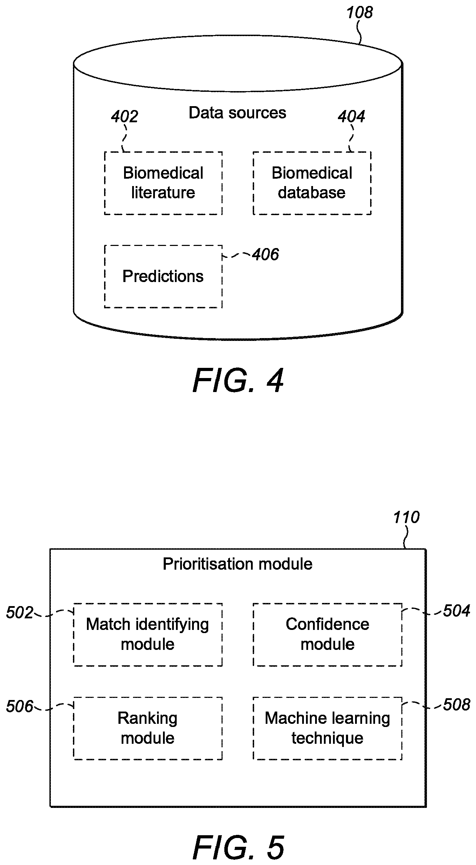

The system 100 comprises a prioritisation module 110 configured to prioritise the biological targets based on the extents of alignment. For example, biological targets aligning well to the classes selected by the user may be considered to match the user requirements and may be prioritised over non-matching biological targets. Alternatively or additionally, biological targets may be prioritised by being ranked in order of closeness to the user requirements and/or in order of a level of confidence in the match between the biological target and the user specified criteria. In this context, prioritisation means any form of organisation, categorisation or labelling of the biological targets based on how well they conform to the user-defined criteria of the user input using the extents of alignment. Details of the prioritisation module 110 are described below in relation to Figure 5.

Finally, the system 100 comprises an output module 112 configured to output a representation of one or more prioritised biological targets 114. This may comprise at least some of the biological targets in a ranked order, for example a top ten biological targets whose properties most closely match the allowable classes specified in the user input 102. Alternatively, all biological targets considered to match the user requirements may be reported, or any other suitable form of reporting of biological targets organised, categorised or labelled by the prioritisation module may be provided.

Accordingly, the present disclosure extends to a system 100 for prioritising biological targets based on a user input. The system 100 comprises: an input module 104 configured to receive the user input, the user input comprising a selection of classes of one or more categories; an analysis module 106 configured, for each of a plurality of biological targets, to determine an extent of alignment of the biological target to each selected class; a prioritisation module 110 configured to prioritise the biological targets based on the extents of alignment; and an output module 112 configured to output a representation of one or more prioritised biological targets. The present disclosure also extends to systems in which the selection of classes of one or more categories is not based on a user input, but rather may, for example, be predetermined or automatically generated.

The present disclosure also extends to a computer-implemented method 200 of prioritising biological targets based on a user input. The method 200 comprises: receiving 202 the user input, the user input comprising a selection of classes of one or more categories; for each of a plurality of biological targets, determining 204 an extent of alignment of the biological target to each selected class; prioritising 206 the biological targets based on the extents of alignment; and outputting 208 a representation of one or more prioritised biological targets. The present disclosure also extends to methods in which the selection of classes of one or more categories is not based on a user input, but rather may, for example, be predetermined or automatically generated.

As indicated above, the analysis module 106 is configured to determine an extent of alignment of each of a plurality of biological targets to each allowable class that has been selected by the user. This creates a measure of how well each of the biological targets conforms to the requirements that have been specified by the user so that the biological targets may subsequently be prioritised based on their level of conformity to the user's requirements. As mentioned above, the extent of alignment between one of the biological targets and a respective allowable class may take the form of a likelihood that that the biological target falls within the respective allowable class.

In an example, a user input 102 may comprise a selection of allowable classes across the categories of safety and biological rationale. In the category of safety, the user may have selected the classes according to which modulation of the biological target is either known not to lead to serious clinical adverse events or not predicted to lead to serious clinical adverse events. In the category of biological rationale, the user may have selected the classes in which dysregulation of the target is either known not to cause disease or predicted not to cause disease. These example user selections of classes are shown in the following Table 1.

**Table 1**

| Category | Class | | User input |
|---|---|---|---|
| Safety | | 1. Modulation of the target is known to lead to serious clinical adverse events. | Not selected |
| | | 2. Modulation of the target is predicted but not known to lead to serious clinical adverse events. | Not selected |
| | | 3. Modulation of the target is not known or predicted to lead to serious clinical adverse events. | Selected |
| | | 4. Modulation of the target is known not to lead to serious clinical adverse events. | Selected |
| Biological Rationale | | 1. Dysregulation of the target is known to cause disease. | Not selected |
| | | 2. Dysregulation of the target is predicted but not known to cause disease. | Not selected |
| | | 3. Dysregulation of the target is not known or predicted to cause disease. | Selected |
| | | 4. Dysregulation of the target is known not to cause disease. | Selected |

In this example, the analysis module 106 is configured to determine the alignment of each of a plurality of biological targets with classes 3 and 4 of the safety category and to determine the alignment of each of the biological targets with classes 3 and 4 of the biological rational category. This may be achieved by reference to one or more data sources containing data relating to the biological targets which may be used to determine the characteristics of the biological targets and to thereby infer how well they conform to the user's selected classes.

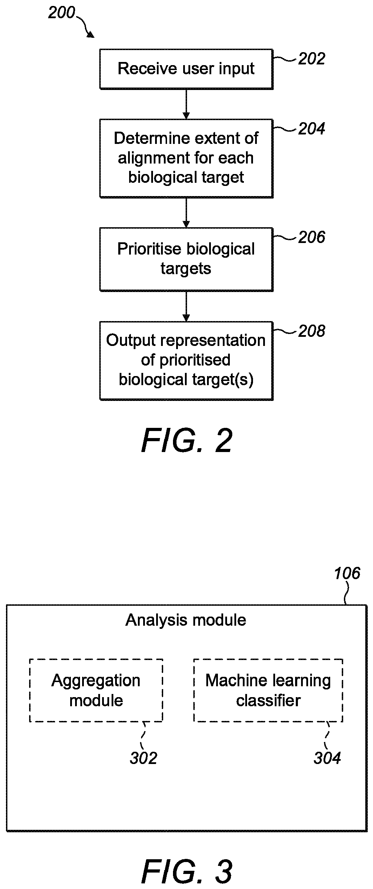

As such, the analysis module 106 may perform the role of a classifier by querying data from one or more data sources to classify each biological target with a likelihood of falling within each class of each category. In suitable examples, the likelihood may take the form of a probability normalised across the classes of a given category. The assignment of likelihoods to each category class may for example be achieved using various probabilistic classifier approaches such as naïve Bayes, logistic regression, or support vector machines. Additionally or alternatively, the extents of alignment between the biological targets and the user requirements may be determined using a machine learning approach, such as a trained machine learning classifier. As such, the analysis module 106 may suitably comprise a machine learning classifier 304 as shown in Figure 3.

If multiple data sources are used, the analysis module 106 may be configured to generate combined likelihoods that take into account multiple data sources. The use of multiple data sources may result in multiple classifications for the same category, and as a result some form of aggregation may be required to return a final classification for a given biological target and a given category. This may be performed in various ways, such as by determining a weighted average of the classifications across all available data sources. In this case, the analysis module 106 may suitably comprise an aggregation module 302 as shown in Figure 3 configured to generate the extents of alignment by aggregating them from classifications based on respective data sources.

Confidence scores may be assigned during the classification to indicate a level of confidence in the determined extents of alignment between the biological targets and the user requirements. The confidence scores could be inferred from a distribution of results from multiple data sources and/or from a machine learning model in the case where the analysis module 106 comprises a machine learning classifier 304 or from any other suitable calculated method.

With reference to Figure 4, it will be appreciated that in the case of the machine learning method, the machine learning classifier 304 may be trained using one or more data sources 108 that may comprise biomedical literature 402, at least one biomedical database 404, and predictions 406 relating to the characteristics and properties of biological targets. In the case of ingesting biomedical literature 402, the machine learning classifier 304 may be configured to examine text of the literature to determine likely classes of a given target for a given category. For example, if a drug target is frequently mentioned in biomedical literature alongside words that indicate severe or serious side effects, then a likely class assigned to this target in the category of safety could relate to a high likelihood of an adverse reaction.

When the analysis module 106 has determined the extents of alignment between the biological targets and the user-selected category classes, this completes the stage of characterising the biological targets. The system 100 is then ready to sort and organise the characterised biological targets by prioritising them according to the extents of alignment in order to return to the user viable suggestions of biological targets that conform to the user's requirements.

Referring to Figure 5, prioritisation module 110 may comprise a match identifying module 502 configured to identify biological targets that are considered to match the user input 102 by applying a minimum required extent of alignment to the respective biological targets for each user-selected class. Suitably, biological targets that are considered to match the user input may be required to meet a minimum required extent of alignment for every selected class. Where matching biological targets are identified, the output module 112 of the system 100 may be configured to output a representation of the biological targets that match the user input, and may additionally be configured to output a list of targets deemed unsuitable based on the user input. These outputs may for example be provided to the user using a graphical user interface.

The prioritisation module 110 may comprise a confidence module 504 configured to determine confidence metrics, for example using a machine learning technique 508, indicating a measure of confidence in the extents of alignment. In this case, the prioritisation module 110 may also comprise a ranking module 506 configured to rank the biological targets, or a subset of the biological targets such as those that match the user input, based on the confidence metrics. The ranking module 506 may additionally or alternatively be included in the prioritisation module 110 to rank some or all of the biological targets based on their extents of alignment to the selected classes. If a ranking and/or confidence metrics are determined, the output module 112 may be configured to output a representation of the ranking or confidence metrics. In embodiments, these may be output to the user using a graphical user interface. It will be appreciated that the confidence metrics may for example be represented as a percentage confidence, a text string (such as High, Medium or Low), or any other suitable method.

In some embodiments, a minimum confidence level that a target belongs to a particular category class may be included in the requirements of the user input 102. Additionally or alternatively, the user input 102 may comprise a user-indication of a relative importance of categories. In this case, the prioritisation module 110 may be configured to prioritise the biological targets using the indication of relative importance. For example, a user may indicate that the category of safety is more important than the category of ligandability.

The output module 112 may be configured to supply further information to the user. For example, the class with the highest likelihood for each category may be provided to the user through a graphical user interface or other means of reporting. Alternatively, an indication in certain instances that insufficient information is available from which to classify a target may be returned to the user. The output module 112 may also be configured to specify to the user that a given target falls within some user defined category classes but not others.

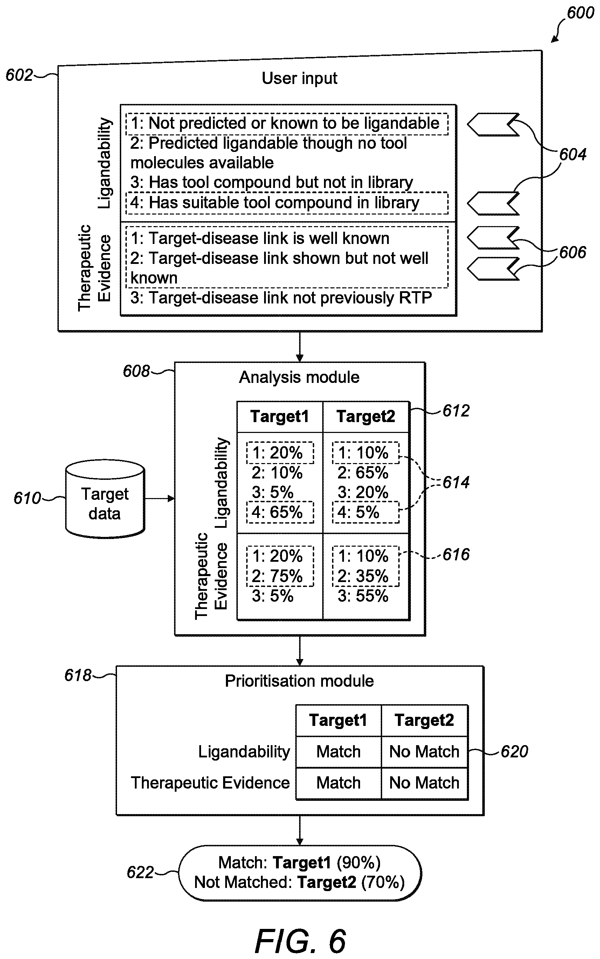

Referring to Figure 6, a non-limiting example use case 600 of an embodiment of the invention is shown. In the example, two categories are supplied from which the user may select suitable classes. The categories are ligandability and therapeutic evidence. In the use case 600 of Figure 6, there is a user input 602 comprising two classes 604 of ligandability that have been selected as suitable by a user. The selected classes of ligandability are class 1: 'Not predicted or known to be ligandable' and class 4: 'Has suitable tool compound in library'. It is to be noted that classes 1 and 4 of ligandability are not mutually adjacent (i.e. are not adjacent each other) in the scale of classes 1 to 4. Rather, they have other classes (2 and 3) between them, and they represent highly opposing classes of the category. In the category of therapeutic evidence, the user has specified that the target fall either within class 1: 'Target-disease link is well known' or within class 2: 'Target-disease link shown but not well known' 606. This requirement may be expressed by saying that the target should be at least shown to be linked to a disease.

After receiving the user input 602, an analysis module 608 examines data from one or more data sources 610 relating to known biological targets in order to classify each one. In the present use case, two targets 612 are considered. The analysis module 608 comprises a classifier which returns percentages indicating the likelihood of each target falling within each class of each category. For example, the determined probability of Target 1 falling within class 1 of the ligandability category is 20% as shown in Figure 6. Similarly, the probability of Target 2 falling within class 3 of the therapeutic evidence category is 55%.

The percentage likelihoods determined by the analysis module 608 may now be used by the prioritisation module to determine how well Targets 1 and 2 conform to requirements stipulated in the user input 602. In the present use case 600, the prioritisation module 618 is configured to determine which of the targets, Targets 1 and 2, are considered to match the requirements of the user input 602. For example, for the category of ligandability, a target must be likely to fall within either class 1 or class 4 in order to be considered to be a match for that category. In the present example use case 600, a minimum threshold of 80% likelihood of falling within one of the selected classes is required. For ligandability, the user selected classes 1 and 4, and the analysis module 608 determined that the likelihood of Target 1 falling within class 1 is 20% and the likelihood of Target 1 falling within class 4 is 65%. As a result, the likelihood of Target 1 falling within one of the acceptable classes is 20% + 65% = 85%. Since this is more than the minimum 80% threshold, Target 1 is considered to match the user requirements for the category of ligandability.

By the same process, Target 2 is 10% + 5% = 15% likely (see reference numeral 614) to fall within one of the acceptable classes for ligandability. Since 15% falls short of the 80% threshold for being considered to be a match, Target 2 is not considered to match the user input 602 for the category of ligandability.

Using the same approach and the same 80% threshold, the prioritisation module 618 computes the percentages 616 for the category of therapeutic evidence and finds that Target 1 is 20% + 75% = 95% likely to fall within an acceptable class and is therefore a match for therapeutic evidence, while Target 2 is 10% + 35% = 45% likely to fall within an acceptable class and is therefore not a match for therapeutic evidence. The prioritisation module 618 thereby produces the result 620 that Target 1 is a match for both categories while Target 2 is a match for neither category. It will be appreciated that in other embodiments or use cases, other thresholds may be used. In some cases, one or more thresholds may be determined automatically using further techniques such as machine learning.

In the use case 600, the analysis module 608 is configured to determine confidence metrics representing confidence levels that that the classifications are accurate for each target. An output module (not shown) is configured to output 622 the matching targets followed by the non-matching targets, each accompanied by their respective confidence score. As shown in the example of use case 600, the output 622 supplies Target 1 as a match with a confidence score of 90% followed by Target 2 as a non-match with a confidence score of 70%.

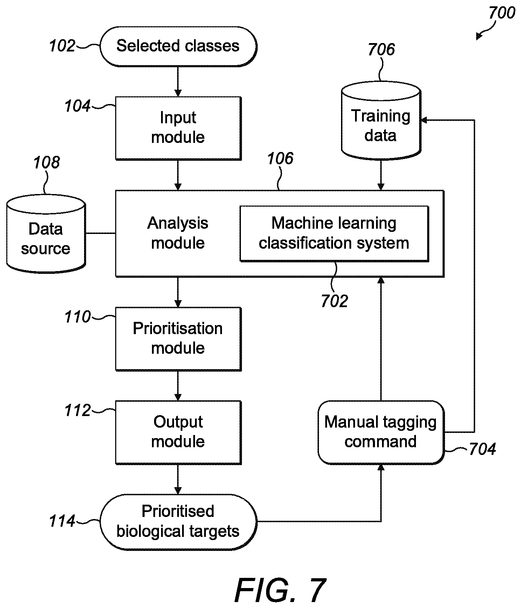

Figure 7 shows a variation 700 of the system 100 of Figure 1. The variation 700 includes a feedback loop to enable manual user feedback to be provided to a machine learning classification system 702 of the analysis module 106. The system 700 comprises a user input means such as a graphical user interface configured to receive a manual tagging command 704 supplied by the user to override at least part of the output 114. For example, an expert in the field may know that a given biological target has a high likelihood of causing adverse side effects, whereas the machine learning classification system 702 may have determined this likelihood as being low. As a result, the user may manually assign a label of 'High' to the biological target for a category of 'Risk of causing adverse side effects', thereby overriding the system's output of 'Low'. Thereafter, this manual input may be fed back into the machine learning classification system 702 automatically and used to further train the classifier. Alternatively or additionally, the manual input 704 may be stored and used in subsequent uses of the system 700 by inclusion in training data 706. Other methods of incorporating user feedback are also envisaged such as the use of techniques such as supervised or semi-supervised approaches in machine learning methods as well as the use of unsupervised machine learning techniques.

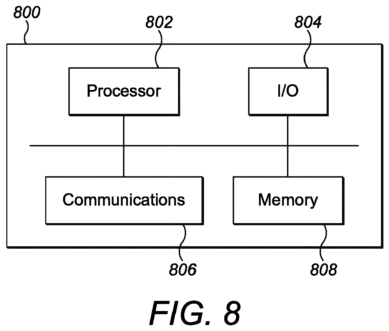

A computer apparatus 800 suitable for implementing methods according to the present invention is shown in Figure 8. The apparatus 800 comprises a processor 802, an input-output device 804, a communications portal 806 and computer memory 808. The memory 808 may store code that, when executed by the processor 802, causes the apparatus 800 to perform the method 200 shown in Figure 2.

In the embodiment described above the server may comprise a single server or network of servers. In some examples the functionality of the server may be provided by a network of servers distributed across a geographical area, such as a worldwide distributed network of servers, and a user may be connected to an appropriate one of the networks of servers based upon a user location.

The above description discusses embodiments of the invention with reference to a single user for clarity. It will be understood that in practice the system may be shared by a plurality of users, and possibly by a very large number of users simultaneously.

The embodiments described above are fully automatic. In some examples a user or operator of the system may manually instruct some steps of the method to be carried out.

In the described embodiments of the invention the system may be implemented as any form of a computing and/or electronic device. Such a device may comprise one or more processors which may be microprocessors, controllers or any other suitable type of processors for processing computer executable instructions to control the operation of the device in order to gather and record routing information. In some examples, for example where a system on a chip architecture is used, the processors may include one or more fixed function blocks (also referred to as accelerators) which implement a part of the method in hardware (rather than software or firmware). Platform software comprising an operating system or any other suitable platform software may be provided at the computing-based device to enable application software to be executed on the device.

Various functions described herein can be implemented in hardware, software, or any combination thereof. If implemented in software, the functions can be stored on or transmitted over as one or more instructions or code on a computer-readable medium. Computer-readable media may include, for example, computer-readable storage media. Computer-readable storage media may include volatile or non-volatile, removable or non-removable media implemented in any method or technology for storage of information such as computer readable instructions, data structures, program modules or other data. A computer-readable storage media can be any available storage media that may be accessed by a computer. By way of example, and not limitation, such computer-readable storage media may comprise RAM, ROM, EEPROM, flash memory or other memory devices, CD-ROM or other optical disc storage, magnetic disc storage or other magnetic storage devices, or any other medium that can be used to carry or store desired program code in the form of instructions or data structures and that can be accessed by a computer. Disc and disk, as used herein, include compact disc (CD), laser disc, optical disc, digital versatile disc (DVD), floppy disk, and bluray disc (BD). Further, a propagated signal is not included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fiber optic cable, twisted pair, DSL, or wireless technologies such as infrared, radio, and microwave are included in the definition of communication medium. Combinations of the above should also be included within the scope of computer-readable media.

Alternatively, or in addition, the functionality described herein can be performed, at least in part, by one or more hardware logic components. For example, and without limitation, hardware logic components that can be used may include Field-programmable Gate Arrays (FPGAs), Program-specific Integrated Circuits (ASICs), Program-specific Standard Products (ASSPs), System-on-a-chip systems (SOCs). Complex Progrmmable Logic Devices (CPLDs), etc.

Although illustrated as a single system, it is to be understood that the computing device may be a distributed system. Thus, for instance, several devices may be in communication by way of a network connection and may collectively perform tasks described as being performed by the computing device.

Although illustrated as a local device it will be appreciated that the computing device may be located remotely and accessed via a network or other communication link (for example using a communication interface).

The term 'computer' is used herein to refer to any device with processing capability such that it can execute instructions. Those skilled in the art will realise that such processing capabilities are incorporated into many different devices and therefore the term 'computer' includes PCs, servers, mobile telephones, personal digital assistants and many other devices.

Those skilled in the art will realise that storage devices utilised to store program instructions can be distributed across a network. For example, a remote computer may store an example of the process described as software. A local or terminal computer may access the remote computer and download a part or all of the software to run the program. Alternatively, the local computer may download pieces of the software as needed, or execute some software instructions at the local terminal and some at the remote computer (or computer network). Those skilled in the art will also realise that by utilising conventional techniques known to those skilled in the art that all, or a portion of the software instructions may be carried out by a dedicated circuit, such as a DSP, programmable logic array, or the like.

It will be understood that the benefits and advantages described above may relate to one embodiment or may relate to several embodiments. The embodiments are not limited to those that solve any or all of the stated problems or those that have any or all of the stated benefits and advantages.

Any reference to 'an' item refers to one or more of those items. The term 'comprising' is used herein to mean including the method steps or elements identified, but that such steps or elements do not comprise an exclusive list and a method or apparatus may contain additional steps or elements.

As used herein, the terms "component" and "system" are intended to encompass computer-readable data storage that is configured with computer-executable instructions that cause certain functionality to be performed when executed by a processor. The computer-executable instructions may include a routine, a function, or the like. It is also to be understood that a component or system may be localized on a single device or distributed across several devices.

Further, as used herein, the term "exemplary" is intended to mean "serving as an illustration or example of something".

Further, to the extent that the term "includes" is used in either the detailed description or the claims, such term is intended to be inclusive in a manner similar to the term "comprising" as "comprising" is interpreted when employed as a transitional word in a claim.

The figures illustrate exemplary methods. While the methods are shown and described as being a series of acts that are performed in a particular sequence, it is to be understood and appreciated that the methods are not limited by the order of the sequence. For example, some acts can occur in a different order than what is described herein. In addition, an act can occur concurrently with another act. Further, in some instances, not all acts may be required to implement a method described herein.

Moreover, the acts described herein may comprise computer-executable instructions that can be implemented by one or more processors and/or stored on a computer-readable medium or media. The computer-executable instructions can include routines, sub-routines, programs, threads of execution, and/or the like. Still further, results of acts of the methods can be stored in a computer-readable medium, displayed on a display device, and/or the like.

The order of the steps of the methods described herein is exemplary, but the steps may be carried out in any suitable order, or simultaneously where appropriate. Additionally, steps may be added or substituted in, or individual steps may be deleted from any of the methods without departing from the scope of the subject matter described herein. Aspects of any of the examples described above may be combined with aspects of any of the other examples described to form further examples without losing the effect sought.

It will be understood that the above description of a preferred embodiment is given by way of example only and that various modifications may be made by those skilled in the art. What has been described above includes examples of one or more embodiments. It is, of course, not possible to describe every conceivable modification and alteration of the above devices or methods for purposes of describing the aforementioned aspects, but one of ordinary skill in the art can recognize that many further modifications and permutations of various aspects are possible. Accordingly, the described aspects are intended to embrace all such alterations, modifications, and variations that fall within the scope of the appended claims.

## Claims

1. A computer-implemented method of prioritising biological targets, the method comprising:
receiving a selection of classes of one or more categories, wherein the categories represent properties of biological targets, wherein the categories comprise one or more of ligandability; safety; therapeutic evidence; biological rationale; target expression; and stratifiability, and wherein the classes of the categories represent values or value ranges of the categories;
for each of a plurality of biological targets, determining an extent of alignment of the biological target to each selected class using a trained machine learning classifier, wherein the extent of alignment between a biological target and a selected class comprises a likelihood of the biological target falling within the selected class, and wherein the plurality of biological targets comprise genes, nucleic acid sequences, proteins, amino acid sequences, protein complexes, and/or biological pathways;
prioritising the biological targets based on the extents of alignment;
outputting a representation of one or more prioritised biological targets;
**characterized in that** a user input tool is provided to enable a user to generate a manual tagging command to override at least part of the output, the manual tagging command specifying whether or not one of the biological targets falls within one of the classes; and
training the classifier based on the manual tagging command.

2. A method according to claim 1, wherein the selected classes of one of the categories are not mutually adjacent.

3. A method according to claim 1 or claim 2, wherein the selection of classes comprises at least two classes of the same category.

4. A method according to any preceding claim, comprising receiving a user input comprising the selection of classes of the one or more categories.

5. A method according to any preceding claim, wherein the likelihood corresponds to a distribution normalised across all classes of the same category.

6. A method according to any preceding claim, comprising determining the extents of alignment from one or more data sources, and/or aggregating the extents of alignment from classifications based on respective data sources.

7. A method according to any preceding claim, wherein prioritising the biological targets comprises identifying biological targets that match the user input by applying a minimum required extent of alignment for each selected class.

8. A method according to any preceding claim, comprising determining confidence metrics for the extents of alignment and optionally ranking the biological targets that match the user input based on the confidence metrics, preferably wherein the method further comprises determining the confidence metrics using a machine learning technique and/or outputting a representation of the confidence metrics.

9. A method according to any preceding claim, wherein prioritising the biological targets comprises ranking the biological targets based on their extents of alignment to the selected classes, and preferably wherein the method further comprises outputting a representation of the ranking.

10. A method according to any preceding claim, wherein the user input comprises an indication of relative importance of the categories and prioritising the biological targets comprises using the indication of relative importance.

11. A method according to any of claims 7 to 10, comprising outputting a representation of the biological targets that match the user input.

12. A method according to any preceding claim, comprising providing a graphical user interface as an input and/or output tool.

13. A method according to any preceding claim, comprising using the override command to augment a set of training data.

14. A computer-readable medium storing code that, when executed by a computer, causes the computer to perform the method of any previous claim.

15. A system for prioritising biological targets, the system comprising:
an input module configured to receive a selection of classes of one or more categories wherein the categories represent properties of biological targets, wherein the categories comprise one or more of ligandability; safety; therapeutic evidence; biological rationale; target expression; and stratifiability, and wherein the classes of the categories represent values or value ranges of the categories;
an analysis module configured, for each of a plurality of biological targets, to determine an extent of alignment of the biological target to each selected class using a trained machine learning classifier, wherein the extent of alignment between a biological target and a selected class comprises a likelihood of the biological target falling within the selected class, and wherein the plurality of biological targets comprise genes, nucleic acid sequences, proteins, amino acid sequences, protein complexes, and/or biological pathways;
a prioritisation module configured to prioritise the biological targets based on the extents of alignment;
an output module configured to output a representation of one or more prioritised biological targets; and
a user input tool configured to enable a user to generate a manual tagging command to override at least part of the output, the manual tagging command specifying whether or not one of the biological targets falls within one of the classes, and wherein the classifier is configured to be trained based on the manual tagging command.

## Patentansprüche

1. Computerimplementiertes Verfahren zum Priorisieren biologischer Targets, wobei das Verfahren Folgendes umfasst:
Empfangen einer Auswahl von Klassen einer oder mehrerer Kategorien, wobei die Kategorien Eigenschaften biologischer Targets darstellen, wobei die Kategorien eine oder mehrere der folgenden Eigenschaften umfassen: Ligandierbarkeit, Sicherheit, therapeutische Evidenz, biologische Begründung, Targetexpression und Stratifizierbarkeit, und wobei die Klassen der Kategorien Werte oder Wertebereiche der Kategorien darstellen;
für jedes der Vielzahl von biologischen Targetn, Bestimmen des Ausmaßes der Übereinstimmung des biologischen Targets mit jeder ausgewählten Klasse unter Verwendung eines trainierten maschinellen Lernklassifizierers, wobei das Ausmaß der Übereinstimmung zwischen einem biologischen Target und einer ausgewählten Klasse die Wahrscheinlichkeit umfasst, dass das biologische Target innerhalb der ausgewählten Klasse liegt, und wobei die Vielzahl von biologischen Targets Gene, Nukleinsäuresequenzen, Proteine, Aminosäuresequenzen, Proteinkomplexe und/oder biologische Wege umfasst;
Priorisieren der biologischen Targets basierend auf den Ausmaßen der Übereinstimmung;
Ausgeben einer Darstellung eines oder mehrerer priorisierter biologischer Targets;
**dadurch gekennzeichnet, dass** ein Benutzereingabetool bereitgestellt wird, das es einem Benutzer ermöglicht, einen manuellen Tagging-Befehl zu generieren, um mindestens einen Teil der Ausgabe zu überschreiben, wobei der manuelle Tagging-Befehl angibt, ob eines der biologischen Targets in eine der Klassen fällt oder nicht; und
Trainieren des Klassifizierers basierend auf dem manuellen Tagging-Befehl.

2. Verfahren nach Anspruch 1, wobei die ausgewählten Klassen einer der Kategorien nicht benachbart sind.

3. Verfahren nach Anspruch 1 oder 2, wobei die Auswahl der Klassen mindestens zwei Klassen derselben Kategorie umfasst.

4. Verfahren nach einem der vorstehenden Ansprüche, umfassend das Empfangen einer Benutzereingabe, umfassend die Auswahl von Klassen der einen oder mehreren Kategorien.

5. Verfahren nach einem der vorstehenden Ansprüche, wobei die Wahrscheinlichkeit einer über alle Klassen derselben Kategorie normalisierten Verteilung entspricht.

6. Verfahren nach einem der vorstehenden Ansprüche, umfassend das Bestimmen der Ausmaße der Übereinstimmung aus einer oder mehreren Datenquellen und/oder das Aggregieren der Ausmaße der Übereinstimmung aus Klassifikationen, die auf den jeweiligen Datenquellen basieren.

7. Verfahren nach einem der vorstehenden Ansprüche, wobei das Priorisieren der biologischen Targets das Identifizieren biologischer Targets umfasst, die mit den Benutzereingaben übereinstimmen, indem ein mindestens erforderliches Ausmaß der Übereinstimmung für jede ausgewählte Klasse angewendet wird.

8. Verfahren nach einem der vorstehenden Ansprüche, umfassend das Bestimmen von Vertrauensmetriken für die Ausmaße der Übereinstimmung und optional das Einordnen der biologischen Targets, die der Benutzereingabe basierend auf den Vertrauensmetriken entsprechen, wobei das Verfahren vorzugsweise weiter das Bestimmen der Vertrauensmetriken unter Verwendung einer maschinellen Lerntechnik und/oder das Ausgeben einer Darstellung der Vertrauensmetriken umfasst.

9. Verfahren nach einem der vorstehenden Ansprüche, wobei das Priorisieren der biologischen Targets das Einordnen der biologischen Targets basierend auf ihrem Ausmaß der Übereinstimmung mit den ausgewählten Klassen umfasst, und wobei das Verfahren vorzugsweise weiter das Ausgeben einer Darstellung der Einordnung umfasst.

10. Verfahren nach einem der vorstehenden Ansprüche, wobei die Benutzereingabe eine Angabe der relativen Bedeutung der Kategorien umfasst und das Priorisieren der biologischen Targets die Verwendung der Angabe der relativen Bedeutung umfasst.

11. Verfahren nach einem der Ansprüche 7 bis 10, umfassend das Ausgeben einer Darstellung der biologischen Targets, die mit der Benutzereingabe übereinstimmen.

12. Verfahren nach einem der vorstehenden Ansprüche, umfassend das Bereitstellen einer grafischen Benutzeroberfläche als Eingabe- und/oder Ausgabewerkzeug.

13. Verfahren nach einem der vorstehenden Ansprüche, umfassend das Verwenden des Überschreibungsbefehls zum Erweitern eines Satzes von Trainingsdaten.

14. Ein computerlesbares Medium, das Code speichert, der, wenn er von einem Computer ausgeführt wird, den Computer dazu veranlasst, das Verfahren eines früheren Anspruchs auszuführen.

15. System zum Priorisieren biologischer Targetstrukturen, wobei das System Folgendes umfasst:
ein Eingabemodul, das so konfiguriert ist, dass es eine Auswahl von Klassen einer oder mehrerer Kategorien empfängt, wobei die Kategorien Eigenschaften biologischer Targets darstellen, wobei die Kategorien eine oder mehrere der folgenden Eigenschaften umfassen: Ligandierbarkeit, Sicherheit, therapeutische Evidenz, biologische Begründung, Targetexpression und Stratifizierbarkeit, und wobei die Klassen der Kategorien Werte oder Wertebereiche dieser Kategorien darstellen;
ein Analysemodul, das für jedes einer Vielzahl von biologischen Targets konfiguriert ist, um unter Verwendung eines trainierten maschinellen Lernklassifizierers das Ausmaß der Übereinstimmung des biologischen Targets mit jeder ausgewählten Klasse zu bestimmen, wobei das Ausmaß der Übereinstimmung zwischen einem biologischen Target und einer ausgewählten Klasse die Wahrscheinlichkeit umfasst, dass das biologische Target innerhalb der ausgewählten Klasse liegt, und wobei die Vielzahl von biologischen Targets Gene, Nukleinsäuresequenzen, Proteine, Aminosäuresequenzen, Proteinkomplexe und/oder biologische Wege umfasst;
ein Priorisierungsmodul, das so konfiguriert ist, dass es die biologischen Targets basierend auf den Ausmaßen der Übereinstimmung priorisiert;
ein Ausgabemodul, das so konfiguriert ist, dass es eine Darstellung eines oder mehrerer priorisierter biologischer Targets ausgibt; und
ein Benutzereingabetool, das so konfiguriert ist, dass ein Benutzer einen manuellen Tagging-Befehl generieren kann, um mindestens einen Teil der Ausgabe zu überschreiben, wobei der manuelle Tagging-Befehl angibt, ob eines der biologischen Targets in eine der Klassen fällt oder nicht, und wobei der Klassifizierer so konfiguriert ist, dass er basierend auf dem manuellen Tagging-Befehl trainiert wird.

## Revendications

1. Procédé mis en œuvre par ordinateur de hiérarchisation de cibles biologiques, le procédé comprenant :
la réception d'une sélection de classes d'une ou plusieurs catégories, dans lequel les catégories représentent des propriétés de cibles biologiques, dans lequel les catégories comprennent une ou plusieurs parmi : ligandabilité ; sécurité ; preuve thérapeutique ; justification biologique ; expression de la cible ; et stratifiabilité, et dans lequel les classes des catégories représentent des valeurs ou des plages de valeurs de ces catégories ;
pour chacune d'une pluralité de cibles biologiques, la détermination d'un degré d'alignement de la cible biologique avec chaque classe sélectionnée à l'aide d'un classificateur d'apprentissage automatique entraîné, dans lequel le degré d'alignement entre une cible biologique et une classe sélectionnée comprend la probabilité que la cible biologique appartienne à la classe sélectionnée, et dans lequel la pluralité de cibles biologiques comprend des gènes, des séquences d'acide nucléique, des protéines, des séquences d'acides aminés, des complexes protéiques et/ou des voies biologiques ;
la hiérarchisation des cibles biologiques sur la base des degrés d'alignement ;
la sortie d'une représentation d'une ou plusieurs cibles biologiques classées par ordre hiérarchique ;
**caractérisé en ce qu'**un outil d'entrée utilisateur est fourni pour permettre à un utilisateur de générer une commande manuelle d'étiquetage pour remplacer au moins une partie de la sortie, la commande manuelle d'étiquetage spécifiant si l'une des cibles biologiques appartient ou non à l'une des classes ; et
l'entraînement du classificateur sur la base de la commande manuelle d'étiquetage.

2. Procédé selon la revendication 1, dans lequel les classes sélectionnées de l'une des catégories ne sont pas adjacentes les unes aux autres.

3. Procédé selon la revendication 1 ou la revendication 2, dans lequel la sélection de classes comprend au moins deux classes de la même catégorie.

4. Procédé selon une quelconque revendication précédente, comprenant la réception d'une entrée utilisateur comprenant la sélection de classes des une ou plusieurs catégories.

5. Procédé selon une quelconque revendication précédente, dans lequel la probabilité correspond à une distribution normalisée sur toutes les classes de la même catégorie.

6. Procédé selon une quelconque revendication précédente, comprenant la détermination des degrés d'alignement à partir d'une ou plusieurs sources de données, et/ou l'agrégation des degrés d'alignement à partir de classifications basées sur des sources de données respectives.

7. Procédé selon une quelconque revendication précédente, dans lequel la hiérarchisation des cibles biologiques comprend l'identification de cibles biologiques qui correspondent à l'entrée utilisateur en appliquant un degré minimal requis d'alignement pour chaque classe sélectionnée.

8. Procédé selon une quelconque revendication précédente, comprenant la détermination d'une métrique de confiance pour les degrés d'alignement et facultativement le classement des cibles biologiques qui correspondent à l'entrée utilisateur sur la base de la métrique de confiance, de préférence dans lequel le procédé comprend en outre la détermination de la métrique de confiance au moyen d'une technique d'apprentissage automatique et/ou la sortie d'une représentation de la métrique de confiance.

9. Procédé selon une quelconque revendication précédente, dans lequel la hiérarchisation des cibles biologiques comprend le classement des cibles biologiques sur la base de leur degré d'alignement avec les classes sélectionnées, et de préférence dans lequel le procédé comprend en outre la sortie d'une représentation du classement.

10. Procédé selon une quelconque revendication précédente, dans lequel l'entrée utilisateur comprend une indication d'importance relative des catégories et la hiérarchisation des cibles biologiques comprend l'utilisation de l'indication d'importance relative.

11. Procédé selon l'une quelconque des revendications 7 à 10, comprenant la sortie d'une représentation des cibles biologiques qui correspondent à l'entrée utilisateur.

12. Procédé selon une quelconque revendication précédente, comprenant la fourniture d'une interface graphique utilisateur en tant qu'outil d'entrée et/ou de sortie.

13. Procédé selon une quelconque revendication précédente, comprenant l'utilisation de la commande de remplacement pour enrichir un ensemble de données d'entraînement.

14. Support lisible par ordinateur stockant un code qui, lorsqu'il est exécuté par un ordinateur, amène l'ordinateur à mettre en œuvre le procédé selon une quelconque revendication précédente.

15. Système de hiérarchisation de cibles biologiques, le système comprenant :
un module d'entrée configuré pour recevoir une sélection de classes d'une ou plusieurs catégories, dans lequel les catégories représentent des propriétés de cibles biologiques, dans lequel les catégories comprennent une ou plusieurs parmi : ligandabilité ; sécurité ; preuve thérapeutique ; justification biologique ; expression de la cible ; et stratifiabilité, et dans lequel les classes des catégories représentent des valeurs ou des plages de valeurs de ces catégories ;
un module d'analyse configuré, pour chacune parmi une pluralité de cibles biologiques, pour déterminer un degré d'alignement de la cible biologique avec chaque classe sélectionnée à l'aide d'un classificateur d'apprentissage automatique entraîné, dans lequel le degré d'alignement entre une cible biologique et une classe sélectionnée comprend la probabilité que la cible biologique appartienne à la classe sélectionnée, et dans lequel la pluralité de cibles biologiques comprend des gènes, des séquences d'acide nucléique, des protéines, des séquences d'acides aminés, des complexes protéiques et/ou des voies biologiques ;
un module de hiérarchisation configuré pour hiérarchiser les cibles biologiques sur la base des degrés d'alignement ;
un module de sortie configuré pour sortir une représentation d'une ou plusieurs cibles biologiques classées par ordre hiérarchique ; et
un outil d'entrée utilisateur configuré pour permettre à un utilisateur de générer une commande manuelle d'étiquetage pour remplacer au moins une partie de la sortie, la commande manuelle d'étiquetage spécifiant si l'une des cibles biologiques appartient ou non à l'une des classes, et dans lequel le classificateur est configuré pour être entraîné sur la base de la commande manuelle d'étiquetage.
